(19) Europäisches Patentamt
European Patent Office
Office européen des brevets

(11)  **EP 4 137 043 B1**

(12)  **EUROPEAN PATENT SPECIFICATION**

(45) Date of publication and mention
of the grant of the patent:
**22.01.2025  Bulletin 2025/04**

(21) Application number: **22190267.9**

(22) Date of filing: **12.08.2022**

(51) International Patent Classification (IPC):
**A61B 5/021** *(2006.01)*     **A61B 5/024** *(2006.01)*
**A61B 5/00** *(2006.01)*

(52) Cooperative Patent Classification (CPC):
**A61B 5/02116; A61B 5/02416; A61B 5/681;
A61B 5/7221;** A61B 5/6816; A61B 2560/0223

(54)  **APPARATUS AND METHOD FOR ESTIMATING BLOOD PRESSURE**

VORRICHTUNG UND VERFAHREN ZUR SCHÄTZUNG DES BLUTDRUCKS

APPAREIL ET PROCÉDÉ POUR ESTIMER LA PRESSION SANGUINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2021  KR 20210108068**

(43) Date of publication of application:
**22.02.2023  Bulletin 2023/08**

(73) Proprietor: **Samsung Electronics Co., Ltd.
Suwon-si, Gyeonggi-do 16677 (KR)**

(72) Inventors:
• **Jang, Dae Geun**
**Yongin-si (KR)**
• **Park, Chang Soon**
**Chungju-si (KR)**
• **Kwon, Ui Kun**
**Hwaseong-si (KR)**
• **Kim, Young Soo**
**Seoul (KR)**

(74) Representative: **Grünecker Patent- und
Rechtsanwälte
PartG mbB
Leopoldstraße 4
80802 München (DE)**

(56) References cited:
**EP-A1- 3 613 340     EP-A1- 3 632 302**

## Description

BACKGROUND

### 1. Field

One or more example embodiments relate to detecting pulse wave signals from a subject, and non-invasively estimating blood pressure of the subject based on the pulse wave signals .

### 2. Description of the Related Art

Research on information technology (IT)-medical convergence technology, in which IT and medical technology are combined, is being recently carried out to address the aging population structure, rapid increase in medical expenses, and shortage of specialized medical service personnel. Particularly, monitoring of the health condition of the human body is not limited to a fixed place, such as a hospital, but is expanding to a mobile healthcare sector for monitoring a user's health status at any time and any place in daily life at home and office.
EP 3,613,340 A1 discloses an apparatus and method for estimating blood pressure for non-invasively estimating blood pressure. The apparatus includes a bio-signal measurer configured to measure a bio-signal from a user and a processor configured to estimate blood pressure using the measured bio-signal. The processor extracts a first feature and a second feature from the bio-signal at an extraction time, estimates changes in the first feature and the second feature which have occurred during a time period between the extraction time and a calibration time at which the first feature and the second feature are calibrated, and estimates a blood pressure based on the changes in the first feature and the second feature.
EP 3,632,302 A1 discloses an apparatus and method for estimating blood pressure. The apparatus for estimating blood pressure includes a bio-signal measurer configured to measure a bio-signal from a user, and a processor configured to extract one or more feature values from the bio-signal, to adjust a combination coefficient for combining the one or more feature values based on a reference value associated with vascular compliance, and to estimate blood pressure based on the adjusted combination coefficient, and the one or more feature values extracted from the bio-signal.

SUMMARY

The invention is claimed in the independent claims. Preferred embodiments are specified in the dependent claims.
According to the present invention, there is provided, an apparatus for estimating blood pressure that comprises a pulse wave sensor configured to measure a pulse wave signal from an object; and a processor configured to: obtain a first feature and a heart rate based on the pulse wave signal, the first feature comprising a ratio between a first amplitude at a first time point and a second amplitude at a second time point in the pulse wave signal, estimate a mean arterial pressure, MAP, and a pulse pressure, PP, based on the first feature and the heart rate, estimate a first blood pressure based on the MAP and the PP, obtain one or more second features based on the pulse wave signal, the one or more second features comprising a ratio between an amplitude at a maximum point of the pulse wave signal and an area of the pulse wave signal, and/or a ratio between an amplitude at a point of a first reflection wave and an amplitude at a point of a propagation wave among component waveforms constituting unit waveforms of the pulse wave signal, and estimate a second blood pressure by using the one or more second features, wherein the processor is further configured to determine reliability in estimating blood pressure based on directions of changes in the first blood pressure and the second blood pressure compared to a calibration time, obtain a third blood pressure based on the determined reliability by using at least one of the first blood pressure and the second blood pressure, and output the obtained third plod pressure as a final blood pressure.
The processor may be further configured to segment the pulse wave signal into a plurality of unit waveforms, and obtain the heart rate by using the plurality of unit waveform.
The processor may be further configured to: normalize the heart rate and the first feature based on a reference heart rate and a reference first feature that are obtained at a calibration time, respectively, obtain a variation in the pulse wave signal based on the normalized heart rate and the normalized first feature, and obtain the MAP based on the variation in the pulse wave signal and a reference MAP that is measured at the calibration time.
The processor may be further configured to obtain the PP by using a reference blood pressure at a calibration time.
The processor may be further configured to obtain the first blood pressure by combining the MAP and the PP.
The processor may be further configured to: normalize the one or more second features based on a reference second feature that is obtained at a calibration time, obtain a variation in the pulse wave signal based on the normalized one or more second features, and estimate the second blood pressure based on the variation in the pulse wave signal and a reference blood pressure at the calibration time.
Upon determining that the reliability is high, the processor may be further configured to obtain one of the first blood pressure and the second blood pressure, which has a greater variation compared to a reference blood pressure that is measured at a calibration time, as the third blood pressure, and upon determining that the reliability is low, the processor may be further configured to obtain an average value of the first blood pressure and the second blood pressure as the third blood pressure.

If a first value, obtained by subtracting a reference blood pressure from the first blood pressure, has a same sign as a second value obtained by subtracting the reference blood pressure from the second blood pressure, the processor determines that the reliability is high, and if not, the processor determines that the reliability is low.

According to another aspect of the present invention, there is provided a method of estimating blood pressure that comprises measuring a pulse wave signal from an object, obtaining a first feature and a heart rate based on the pulse wave signal, the first feature comprising a ratio between a first amplitude at a first time point and a second amplitude at a second time point in the pulse wave signal, estimating a mean arterial pressure, MAP, and a pulse pressure, PP, based on the first feature and the heart rate, estimating (a first blood pressure based on the MAP and the PP, obtaining one or more second features based on the pulse wave signal, the one or more second features comprising a ratio between an amplitude at a maximum point of the pulse wave signal and an area of the pulse wave signal, and/or a ratio between an amplitude at a point of a first reflection wave and an amplitude at a point of a propagation wave among component waveforms constituting unit waveforms of the pulse wave signal, estimating a second blood pressure based on the one or more second features, determining reliability in estimating blood pressure based on directions of changes in the first blood pressure and the second blood pressure compared to a calibration time, obtaining a third blood pressure based on the reliability by using at least one of the first blood pressure and the second blood pressure, and outputting the obtained third plod pressure as a final blood pressure.

The estimating of the MAP may include: normalizing the heart rate and the first feature based on a reference heart rate and a reference first feature that are obtained at a calibration time, respectively; obtaining a variation in the pulse wave signal based on the normalized heart rate and the normalized first feature; and obtaining the MAP based on the variation in the pulse wave signal and a reference MAP that is measured at the calibration time.

The estimating of the second blood pressure may include: normalizing the obtained one or more second features based on a reference second feature that is obtained at a calibration time; obtaining a variation in the pulse wave signal based on the normalized one or more second features; and estimating the second blood pressure based on the variation in the pulse wave signal and a reference blood pressure at the calibration time.

The obtaining of the third blood pressure may include, in response to determining that the reliability is high, obtaining one of the first blood pressure and the second blood pressure, which has a greater variation compared to a reference blood pressure that is measured at a calibration time, as the third blood pressure, and in response to determining that the reliability is low, obtaining an average value of the first blood pressure and the second blood pressure as the third blood pressure.

The determining of the reliability may include, if a first value, obtained by subtracting a reference blood pressure from the first blood pressure, has a same sign as a second value obtained by subtracting the reference blood pressure from the second blood pressure, determining that the reliability is high, and if not, determining that the reliability is low.

According to another aspect of the present invention, there is provided a wearable device having mounted the above-defined apparatus for estimating blood pressure.

BRIEF DESCRIPTION OF THE DRAWINGS

The above and/or other aspects will be more apparent by describing certain example embodiments, with reference to the accompanying drawings, in which:

FIG. 1 is a block diagram illustrating an apparatus for estimating blood pressure according to an example embodiment of the present disclosure;
FIG. 2 is a diagram explaining the principle of generating component waveforms included in unit waveforms of a pulse wave signal;
FIG. 3 is a block diagram illustrating a configuration of a processor according to an example embodiment of the present disclosure;
FIG. 4 is a block diagram illustrating a configuration of a processor according to another example embodiment of the present disclosure;
FIG. 5 is a block diagram illustrating an apparatus for estimating blood pressure according to another example embodiment of the present disclosure;
FIG. 6 is a flowchart illustrating a method of estimating blood pressure according to an example embodiment of the present disclosure;
FIG. 7 is a flowchart illustrating a method of estimating blood pressure according to another example embodiment of the present disclosure;
FIG. 8 is a flowchart illustrating an example of obtaining a third blood pressure in FIG. 7;
FIG. 9 is a flowchart illustrating another example of obtaining a third blood pressure in FIG. 7; and
FIGS. 10 to 12 are block diagrams illustrating various structures of an electronic device including an apparatus for estimating blood pressure.

DETAILED DESCRIPTION

Example embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

It will be understood that, although the terms "first," "second," etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as "unit" or "module", etc., should be understood as a unit for performing at least one function or operation and that may be embodied as hardware, software, or a combination thereof.

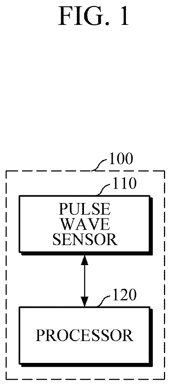

FIG. 1 is a block diagram illustrating an apparatus for estimating blood pressure according to an example embodiment of the present disclosure.

Various examples of the apparatus for estimating blood pressure may be mounted in an electronic device, such as a smartphone, a tablet PC, a desktop computer, a laptop computer, and a wearable device including a wristwatch-type wearable device, a bracelet-type wearable device, a wristband-type wearable device, a ring-type wearable device, a glasses-type wearable device, a headband-type wearable device, and the like.

Referring to FIG. 1, an apparatus 100 for estimating blood pressure includes a pulse wave sensor 110 and a processor 120.

The pulse wave sensor 110 measures pulse wave signals, including a photoplethysmogram (PPG) signal and/or an electrocardiography (ECG), from an object. The pulse wave sensor 110 may include a PPG sensor and an ECG sensor to estimate blood pressure of a human body by analyzing a form of a pulse wave signal that reflects a cardiovascular state. In particular, the object may be a body part that may come into contact with the pulse wave sensor 110, and may be, for example, a body part where pulse waves may be easily measured. For example, the object may be a skin area of the wrist that is adjacent to the radial artery and a skin area of the human body where venous blood or capillary blood passes. However, the object is not limited to the above examples, and may be a peripheral part of the human body, such as a finger, a toe, or the like, blood vessels are densely distributed in the human body.

The pulse wave sensor 110 may include one or more light sources for emitting light toward an object and one or more detectors for detecting light scattered or reflected from or transmitted into the object after light is emitted by the light sources. The light source may include a light emitting diode (LED), a laser diode (LD), a phosphor, and the like. The plurality of light sources may emit light of one or more wavelengths (e.g., green, red, blue, and infrared wavelengths). The detector may include one or more photodiodes, photo transistors (PTr), image sensors (e.g., complementary metal-oxide semiconductor (CMOS) image sensor), etc., but is not limited thereto.

The processor 120 may be electrically or functionally connected to the pulse wave sensor 110 and may control the pulse wave sensor 110 to acquire a pulse wave signal. Upon receiving the pulse wave signal from the pulse wave sensor 110, the processor 120 may perform preprocessing, such as removing noise from the received pulse wave signal. For example, the processor 120 may perform signal correction, such as filtering (e.g., band-pass filtering between 0.4 Hz and 10 Hz), amplification of the pulse wave signal, converting the signal into a digital signal, smoothing, ensemble averaging of continuously measured pulse wave signals, and the like.

The processor 120 estimates blood pressure by analyzing a waveform of the pulse wave signal measured at a blood pressure estimation time. Hereinafter, the term "blood pressure" may refer to any one or both of diastolic blood pressure (DBP) and systolic blood pressure (SBP), unless indicated otherwise.

For example, the processor 120 may obtain a pulse wave signal that is continuously measured during a predetermined period of time, and may segment the pulse wave signal into a plurality of unit waveforms according to cycles of the pulse wave signal. The processor 120 may determine, as a representative waveform, any one of the unit waveforms or a waveform generated by combining two or more of the plurality of unit waveforms, and may estimate blood pressure by using the determined representative waveform.

For example, among the plurality of unit waveforms, the processor 120 may determine, as the representative waveform, a unit waveform having a highest amplitude at a maximum point or a waveform formed by superposition of unit waveforms having an amplitude at a maximum point that is greater than or equal to a threshold value. In another example, the processor 120 may determine, as the representative waveform, an ensemble average of unit waveforms, having a highest average value of similarities between the plurality of unit waveforms, or an ensemble average of unit waveforms having an average value of similarities between the plurality of unit waveforms that is greater than or equal to a threshold value. However, the representative waveform is not limited thereto. In particular, various similarity calculation algorithms may be used, including Euclidean distance, Manhattan Distance, Cosine Distance, Mahalanobis Distance, Jaccard Coefficient, Extended Jaccard Coefficient, Pearson's Correlation Coefficient, Spearman's Correlation Coefficient, and the like.

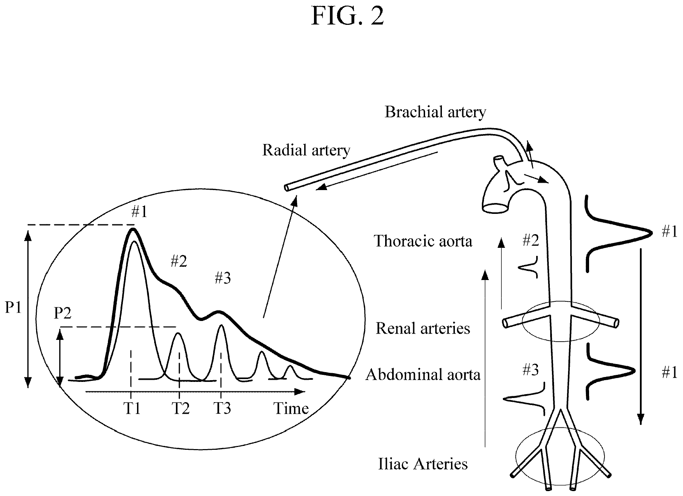

FIG. 2 is a diagram explaining the principle of generating component waveforms included in unit waveforms of a pulse wave signal.

Referring to FIG. 2, a pulse wave signal may be constituted with one propagation wave and a plurality of reflection waves. Specifically, a pulse wave signal may be a summation of a propagation wave #1 propagating from the heart by blood ejection from the left ventricle to peripheral parts of the body or branching points in the blood vessels, and reflection waves #2 and #3 returning from the peripheral parts of the body or the branching points in the blood vessels. The propagation wave #1 is related to cardiac characteristics, and the reflection waves #2 and #3 are related to vascular characteristics. Generally, the propagation wave #1 generated by blood ejection from the left ventricle is mainly reflected from the renal arteries and iliac arteries, to generate the first reflection wave #2 and the second reflection wave #3. As described above, by dividing the unit waveforms of the pulse wave signal into the respective component waveforms #1, #2, and #3, and by analyzing time points T1, T2, and T3 associated with the component waveforms #1, #2, and #3 and/or amplitudes P1, P2, and P3 of the pulse wave signal, etc., the processor 120 may measure blood pressure.

The processor 120 estimates mean arterial pressure (MAP) and pulse pressure (PP) by analyzing the waveform of the pulse wave signal, and estimates blood pressure based on the estimated MAP and PP.

Generally, a variation in MAP is generally proportional to cardiac output (CO) and total peripheral resistance (TPR), as shown in the following Equation 1.

[Equation 1]

$$\Delta \mathrm{MAP} = \mathrm{CO} \times \mathrm{TPR}$$

Herein, ΔMAP denotes a difference in MAP between the left ventricle and the right atrium, in which MAP of the right atrium is generally in a range of 3 mmHg to 5 mmHg, such that the MAP in the right atrium is similar to the MAP in the left ventricle or MAP of the upper arm. If absolute actual CO and TPR values are known, MAP may be obtained from the aorta or the upper arm. However, it may be difficult to estimate absolute CO and TPR values based on a pulse wave signal.

Accordingly, the processor 120 may extract a feature related to cardiac output (CO) (hereinafter referred to as a "CO feature") and a feature related to total peripheral resistance (TPR) (hereinafter referred to as a "TPR feature") from the pulse wave signal, and may obtain MAP by using the CO feature and the TPR feature. Here, the CO feature may be a feature value which shows an increasing trend or a decreasing trend in proportion to an actual CO value, wherein the actual CO value tends to change more than an actual TPR value in a non-stable state.. Further, the TPR feature may be a feature value which shows an increasing/decreasing trend in proportion to an actual TPR value, wherein the actual TPR value tends to change more than an actual CO value in a non-stable state.

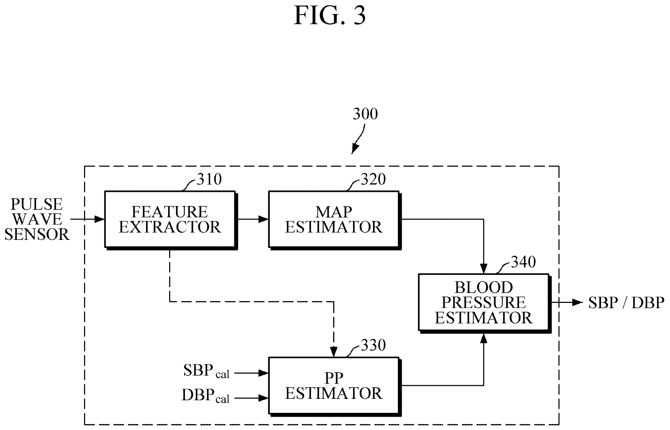

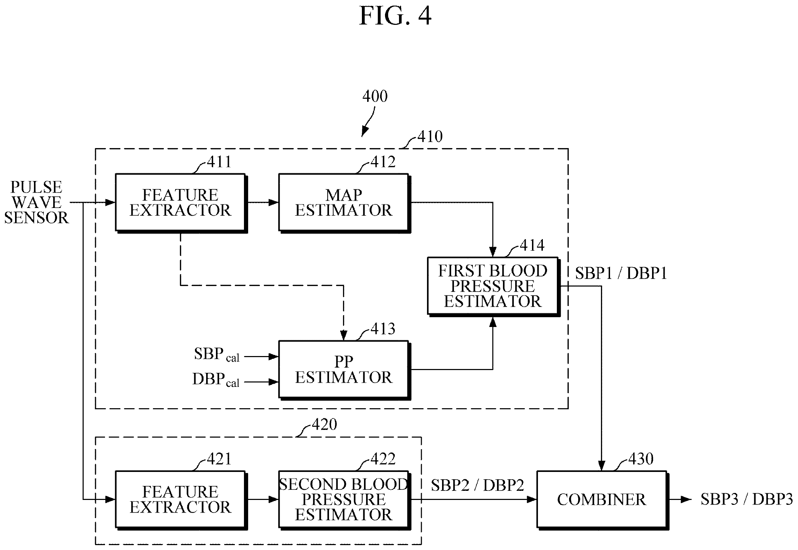

Hereinafter, various examples of estimating blood pressure by the processor 120 will be described with reference to FIGS. 3 and 4.

FIG. 3 is a block diagram illustrating a configuration of a processor according to an example embodiment of the present disclosure.

Referring to FIG. 3, a processor 300 according to an example embodiment of the present disclosure includes a feature extractor 310, an MAP estimator 320, a PP estimator 330, and a blood pressure estimator 340.

The feature extractor 310 may analyze a representative waveform of a pulse wave signal to extract a CO feature and a TPR feature for estimating MAP.

For example, as described above, the feature extractor 310 may obtain a heart rate (HR) by analyzing cycles of unit waveforms of the pulse wave signal measured continuously during a predetermined period of time, and may obtain the obtained heart rate as the CO feature.

The feature extractor 310 may obtain, as the TPR feature, a value SVTPR obtained by multiplying SV and TPR (e.g., a ratio between an amplitude at a first time point and an amplitude at a second time point in the representative waveform of the pulse wave signal). The first time point and the second time point may be associated with, for example, the time point T1 of the propagation wave #1 and the time point T2 of the reflection wave #2, respectively, in the above component waveforms #1, #2, and #3. In particular, the TPR feature may represent a ratio (e.g., P2/P1) between the amplitudes P1 and P2 corresponding to the time point T1 of the propagation wave #1 and the time point T2 of the reflection wave #2. In addition, the first time point and the second time point may be time points predefined to be commonly applied to a plurality of users. However, the time points are not limited thereto, and a personalized time point in which user characteristics are reflected may also be used.

Examples of the CO feature and the TPR feature are described above, but the features are not limited thereto. For example, the feature extractor 310 may obtain, as the CO feature and the TPR feature, a value obtained by using one or an appropriate combination of two or more of time points and amplitudes of the component waveforms of the pulse wave signal, a shape of the waveform of the pulse wave signal, a time point and an amplitude at a maximum point of the pulse wave signal, a time point and an amplitude at a minimum point of the pulse wave signal, an area of the entire region of the waveform of the pulse wave signal, an area of a partial region (e.g., systolic region, diastolic region) of the waveform of the pulse wave signal, and an elapsed time of the pulse wave signal.

The MAP estimator 320 may obtain the CO feature and the TPR feature from the feature extractor 310, and may estimate MAP based on the CO feature and the TPR feature. For example, the MAP estimator 320 may normalize the extracted CO feature and TPR feature by using a CO feature and a TPR feature, which are obtained at a calibration time (e.g., at a resting state), and may obtain a variation in MAP by linearly or nonlinearly combining the normalized values. In addition, the MAP estimator 320 may obtain MAP based on the variation in MAP and a reference MAP measured at the calibration time.

The following Equation 2 is an example of normalizing the CO feature and the TPR feature.

[Equation 2]

$$\Delta feat\_CO = \left( \frac{feat\_CO\_est}{feat\_CO\_cal} - 1 \right)$$

$$\Delta feat\_TPR = \left( \frac{feat\_TPR\_est}{feat\_TPR\_cal} - 1 \right)$$

Herein, $\Delta feat\_CO$ denotes a normalized value of the CO feature, i.e., a variation in CO feature compared to the calibration time; feat_CO_est denotes a CO feature at a blood pressure estimation time; feat_CO_cal denotes the CO feature at the calibration time; $\Delta feat\_TPR$ denotes a normalized value of the TPR feature, i.e., a variation in TPR feature compared to the calibration time; feat_TPR_est denotes a TPR feature at a blood pressure estimation time; feat_TPR_cal denotes the TPR feature at the calibration time.

The following Equation 3 is an example of calculating MAP.

[Equation 3]

$$\Delta MAP = f(\Delta feat\_CO, \Delta feat\_TPR)$$

$$MAP_{est} = \Delta MAP + MAP_{cal}$$

Herein, $\Delta MAP$ denotes the variation in MAP; $\Delta feat\_CO$ and $\Delta feat\_TPR$ denote the normalized value of the CO feature and the normalized value of the TPR feature, respectively; f($\Delta feat\_CO$, $\Delta feat\_TPR$) denotes a predefined linear/non-linear function for combining the normalized values of the CO feature and the TPR feature; $MAP_{est}$ denotes an estimated MAP value; and $MAP_{cal}$ denotes a reference MAP at the calibration time.

The PP estimator 330 may estimate pulse pressure at a current time. For example, the PP estimator 330 may use, as the pulse pressure at the current time, a reference pulse pressure at the calibration time as it is. For example, the PP estimator 330 may obtain the pulse pressure at the current time by using a reference blood pressure measured at the calibration time, i.e., a reference systolic blood pressure and a reference diastolic blood pressure. In particular, the reference blood pressure may be a blood pressure value measured by a device, such as a cuff sphygmomanometer, or by the apparatus 100, at the calibration time when a user is at rest. The following Equation 4 is an example of obtaining pulse pressure.

[Equation 4]

$$PP_{est} = SBP_{cal} - DBP_{cal}$$

Herein, SBPcal and DBPcal denote systolic blood pressure and diastolic blood pressure at the calibration time, respectively; PPest denotes an estimated pulse pressure value at the current time, and the reference pulse pressure value (SBPcal - DBPcal) at the calibration time may be obtained as the obtained pulse pressure value at the current time.

In another example, the PP estimator 330 may estimate the pulse pressure at the current time by using a feature related to pulse pressure (hereinafter referred to as a PP feature) extracted from the pulse wave signal. For example, the PP estimator 330 may normalize the PP feature by using a PP feature at the calibration time, and may obtain a variation in pulse pressure by using the normalized value. In addition, the PP estimator 330 may estimate the pulse pressure at the current time by combining the obtained variation in pulse pressure and the reference pulse pressure at the calibration time using a predefined equation.

In this case, the feature extractor 310 may extract, as the PP feature, one or a combination of two or more of the above various information items which may be obtained by analyzing the waveform of the pulse wave signal. For example, the feature extractor 310 may extract, as the PP feature, a ratio between an area of a systolic phase and an area of a diastolic phase or a ratio between a time interval of the systolic phase and a time interval of the diastolic phase in the representative waveform of the pulse wave signal, but the PP feature is not limited thereto. In this case, the systolic phase may refer to a region from the beginning of the representative waveform to the point of the dicrotic notch (DN) and a region from the point of the dicrotic notch (DN) to an end point.

The blood pressure estimator 340 estimates blood pressure by using the obtained MAP and PP. For example, the blood pressure estimator 340 may independently obtain diastolic blood pressure and systolic blood pressure by applying the MAP and PP to a diastolic blood pressure equation and a systolic blood pressure equation which are defined as linear/non-linear equations. The following Equation 5 is an example of the diastolic blood pressure equation and the systolic blood pressure equation.

[Equation 5]

$$SBP_{est} = MAP_{est} + (1 - \alpha)PP_{est}$$

$$DBP_{est} = MAP_{est} - \alpha PP_{est}$$

Herein, SBPest and DBPest denote the estimated SBP and DBP values, respectively; MAPest and PPest denote the estimated MAP and PP values, respectively; and $\alpha$ is a predefined value.

FIG. 4 is a block diagram illustrating a configuration of a processor according to the present invention.

Two or more blood pressure values are estimated by using two or more algorithm modules, and a final blood pressure value is obtained based on a direction of change in the respective estimated blood pressure values.

Referring to FIG. 4, a processor 400 includes a first algorithm module 410 for obtaining a first blood pressure, a second algorithm module 420 for obtaining a second blood pressure, and a combiner 430 for obtaining a third blood pressure by using the first blood pressure and the second blood pressure.

The first algorithm module 410 includes a feature extractor 411, an MAP estimator 412, a PP estimator 413, and a first blood pressure estimator 414. The respective components of the first algorithm module 410 are described above with reference to FIG. 3, such that a detailed description thereof will be omitted.

The second algorithm module 420 includes a feature extractor 421 and a second blood pressure estimator 422. The second algorithm module 420 uses a feature extraction method and a blood pressure estimation method, which are different from those of the first algorithm module 410 configured to estimate blood pressure based on analysis of a MAP and a PP of a pulse wave signal. A blood pressure estimation result of the first algorithm module 410 is used to evaluate the reliability of a blood pressure estimation result of the second algorithm module 410, and to be combined with the blood pressure estimation result of the second algorithm module 410, to improve accuracy of the blood pressure estimation.

The feature extractor 421 may extract various characteristic points by using a pulse wave signal, a derivative signal and an integrated signal of the pulse wave signal, and the like. For example, by analyzing the waveform of the pulse wave signal, the feature extractor 421 may obtain a variety of information, such as a shape of the waveform, a time point and an amplitude at a maximum point of the pulse wave signal, a time point and an amplitude at a minimum point of the pulse wave signal, an area of the entire region of the pulse wave signal, an area of a partial region (e.g., systolic region, diastolic region) of the pulse wave signal, an elapsed time of the pulse wave signal, and the like. In addition, by obtaining, for example, a second derivative signal of the pulse wave signal and by detecting a local minimum point of the second derivative signal, the feature extractor 421 may obtain time and amplitude information associated with component waveforms constituting the unit waveforms of the pulse wave signal.

Upon obtaining a variety of information from the pulse wave signal, the feature extractor 421 may obtain one or more features related to blood pressure by using one or a combination of two or more of the obtained information. In particular, the feature extractor 421 may obtain the same features for DBP and SBP or may obtain different features for each of DBP and SBP. According to the invention, the feature extractor 421 extracts, as the features, a ratio between an amplitude at a maximum point of the pulse wave and an area of the pulse wave signal, and/or a ratio between an amplitude at a point of the first reflection wave and an amplitude at a point of the propagation wave among the component waveforms constituting the unit waveforms of the pulse wave signal, and the like.

The second blood pressure estimator 422 estimates a second blood pressure by using the features obtained by the feature extractor 421.

For example, the second blood pressure estimator 422 may normalize a feature at the current estimation time by using a feature at the calibration time as shown in the above Equation 2, may obtain a variation in second blood pressure (DBP variation and SBP variation) by using the normalized value, and may obtain the second blood pressure (DBP and SBP) by combining the second blood pressure variation and the reference blood pressure (reference DBP and reference SBP) at the calibration time. The following Equation 6 is an example of an equation for calculating the variation in second blood pressure and the second blood pressure, but is not limited thereto.

[Equation 6]

$$\Delta BP = f(\Delta feat\_1, \cdots, \Delta feat\_n)$$

$$BP_{est} = \Delta BP + BP_{cal}$$

Herein, $\Delta feat\_1$,..., and $\Delta feat\_n$ denote normalized values of n number of features, in which n is a natural number greater than 1; and f($\Delta feat\_1$,..., and $\Delta feat\_n$) is a predefined linear/non-linear equation for combining the normalized values of the n number of features and may be defined differently for each of DBP and SBP; $\Delta BP$

denotes the variation in second blood pressure; BPest denotes an estimated second blood pressure value; and BPcal denotes the reference blood pressure at the calibration time.

In another example, the second blood pressure estimator 422 may obtain SBP in the second blood pressure based on the SBP variation as described above, and may obtain DBP based on a variation in MAP. For example, the second blood pressure estimator 422 may normalize a feature for SBP by using a feature for SBP at the calibration time, and may obtain the SBP variation and SBP by combining the features as shown in the above Equation 6.

In addition, the second blood pressure estimator 422 may normalize a feature related to MAP (hereinafter referred to as MAP feature) and a PP feature, which are extracted by the feature extractor 421, by using MAP and PP features at the calibration time, and may obtain a variation in MAP and a variation in PP by combining the normalized values. According to the invention, the MAP feature includes a ratio between a maximum amplitude value and an area of an entire region or a partial region (e.g., systolic region or diastolic region) of the pulse wave signal, and/or a ratio between an amplitude at a point of the reflection wave and an amplitude at a point of the propagation wave. The PP feature may include a ratio between areas of the systolic region and the diastolic region, and/or a ratio between time intervals of the systolic region and the diastolic region, etc., but the PP feature is not limited thereto. The second blood pressure estimator 422 may estimate DBP by combining the obtained variation in MAP, variation in PP, and the reference DBP as shown in the following Equation 7. However, Equation 7 is merely an example.

[Equation 7]

$$DBP_{est} = (\Delta MAP - \alpha \Delta PP) + DBP_{cal}$$

Herein, DBPest denotes the estimated SBP value; $\Delta MAP$ denotes the variation in MAP; $\Delta PP$ denotes the variation in PP; $DBP_{cal}$ denotes the reference DBP at the calibration time; and $\alpha$ is a predefined value.

The combiner 430 obtains a third blood pressure by using the first blood pressure and the second blood pressure. According to the invention, the combiner 430 determines reliability in estimating blood pressure by using the first blood pressure and the second blood pressure, and obtains the third blood pressure by using any one or a combination of two or more of the first blood pressure and the second blood pressure based on the determined reliability.

According to the invention, the combiner 430 determines the reliability based on a direction of change in the first blood pressure and a direction of change in the second blood pressure. For example, if the first blood pressure and the second blood pressure show the same directions of change, i.e., if a first value, obtained by subtracting the reference blood pressure from the first blood pressure, has the same sign as a second value obtained by subtracting the reference value from the second blood pressure, the combiner 430 may determine that the reliability is high, and if the directions of change are different, i.e., if the first value and the second value have different signs, the combiner 430 may determine that the reliability is low.

In another example, the combiner 430 may determine the reliability further based on a magnitude of a blood pressure variation, in addition to the direction of change in blood pressure. For example, even when determining that the reliability is low based on different directions of change in blood pressure, if there is a slight difference between a magnitude of the first value (absolute value of the first value) and a magnitude of the second value (absolute value of the second value), i.e., if a difference between a magnitude of the first value and a magnitude of the second value is less than or equal to a predetermined threshold value, the combiner 430 may determine again that the reliability is high.

The combiner 430 may obtain the third blood pressure based on the reliability according to predetermined criteria. For example, if the reliability is high, the combiner 430 may obtain one of the first blood pressure and the second blood pressure, which has a greater variation, as the third blood pressure; and if the reliability is low, the combiner 430 may obtain a statistical value (e.g., average value) of the first blood pressure and the second blood pressure as the third blood pressure. Alternatively, if the reliability is high, the combiner 430 may obtain the first blood pressure (or the second blood pressure) as the third blood pressure; and if the reliability is low, the combiner 430 may obtain the second blood pressure (or the first blood pressure) as the third blood pressure value. Alternatively, if the reliability is high, the combiner 430 may obtain the first blood pressure (or the second blood pressure) as the third blood pressure; and if the reliability is low, the combiner 430 may obtain a statistical value (e.g., average value) of the first blood pressure and the second blood pressure as the third blood pressure. Alternatively, if the reliability is high, the combiner 430 may obtain one of the first blood pressure and the second blood pressure, which has a greater variation, as the third blood pressure; and if the reliability is low, the combiner 430 may obtain any one of the first blood pressure and the second blood pressure as the third blood pressure. Alternatively, if the reliability is high, the combiner 430 may obtain one of the first blood pressure and the second blood pressure, which has a greater variation, as the third blood pressure; and if the reliability is low, the combiner 430 may obtain one of the first blood pressure and the second blood pressure, which has a smaller variation, as the third blood pressure. However, these examples may be changed variously.

The combiner 430 may exclude a blood pressure value, having a small variation, from the determination of the reliability. For example, if a magnitude of the second value (or the first value) is less than a predetermined magnitude, the combiner 430 may exclude the second value (or the first value), and may obtain the first blood pressure (or the second blood pressure) as the third blood pressure. In particular, if a blood pressure value, remaining after excluding any one of the first blood pressure and the second blood pressure, is not a blood pressure value estimated by a predetermined main algorithm module, or if both of the first blood pressure and the second blood pressure are less than a predetermined magnitude, the combiner 430 may guide a user to measure blood pressure again or to perform calibration again.

The above description is given of an example of estimating two blood pressure values by using two algorithm modules 410 and 420, and estimating a final blood pressure based on the two blood pressure values. However, the present disclosure is not limited thereto.

For example, the processor 400 may include three or more predefined algorithm modules and the combiner 430. Each of the three or more algorithm modules may estimate blood pressure by using their own predefined methods. The combiner 430 may determine reliability based on estimated three or more directions of change in blood pressure, and may obtain a final blood pressure based on the reliability according to predefined criteria. For example, when estimating three blood pressure values, if all the three blood pressure values show the same directions of change, the combiner 430 may determine that the reliability is high, and may select a blood pressure value having a largest variation as the final blood pressure. Alternatively, if two of the three blood pressure values show the same directions of change, the combiner 430 may determine that the reliability is medium, and by applying a predetermined weight to the two blood pressure values showing the same directions of change, the combiner 430 may calculate an average value of all the blood pressure values or may calculate an average value of only the two blood pressure values showing the same directions of change to determine the final blood pressure. There may be various other criteria for determining reliability according to the number of algorithm modules and criteria for determining blood pressure based on the reliability.

The first algorithm module 410, the second algorithm module 420, and the combiner 430 of the processor 120 may process a pulse wave signal to estimate blood pressure in real time while the pulse wave sensor 110 continuously measures the pulse wave signal from the user.

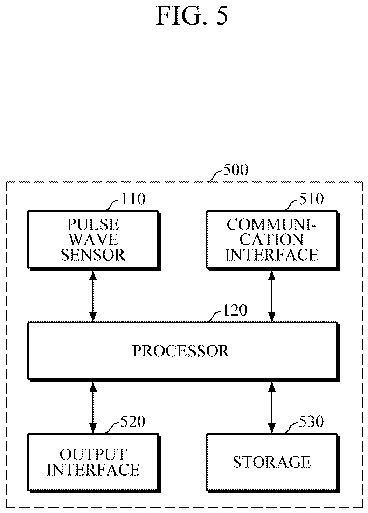

FIG. 5 is a block diagram illustrating an apparatus for estimating blood pressure according to another example embodiment of the present disclosure.

Referring to FIG. 5, an apparatus 500 for estimating blood pressure includes the pulse wave sensor 110, the processor 120, a communication interface 150, an output interface 520, and a storage 530. The pulse wave sensor 110 and the processor 120 are described in detail above, such that a description thereof will be omitted.

The communication interface 510 may be electrically or functionally connected to the processor 120, and may communicate with an external electronic device under the control of the processor 120 by using various communication techniques to transmit and receive necessary data, e.g., reference blood pressure, various blood pressure estimation equations, blood pressure estimation results, and the like. The external electronic device may include a smartphone, a tablet PC, a desktop computer, a laptop computer, a wearable device, etc., but is not limited thereto. The communication techniques may include Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, 3G, 4G, and 5G, communications, and the like. However, the communication techniques are not limited thereto.

The output interface 520 may output processing results of the pulse wave sensor 110 and/or the processor 120. The output interface 520 may provide a user with information by various visual/non-visual methods using a visual output module including a display, an audio output module such as a speaker, or a haptic module using vibrations, tactile sensation, and the like.

The storage 530 may store data required for the pulse wave sensor 110 and/or the processor 120, and/or the processing results of the pulse wave sensor 110 and/or the processor 120. For example, the storage 530 may store blood pressure estimation equations, criteria for determining reliability, user characteristics (e.g., gender, age, health condition, etc.), the pulse wave signal, features, and reference blood pressure, which are obtained during calibration, and the pulse wave signal, features, and estimated blood pressure values, which are generated at the blood pressure estimation time, and the like. The information of the user characteristics may be input by the user (for example, by using a screen touch-based user interface or an input device such as a keyboard) to be stored in the storage 530. The information of the user characteristics may be also referred to as user profile information.

The storage 530 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

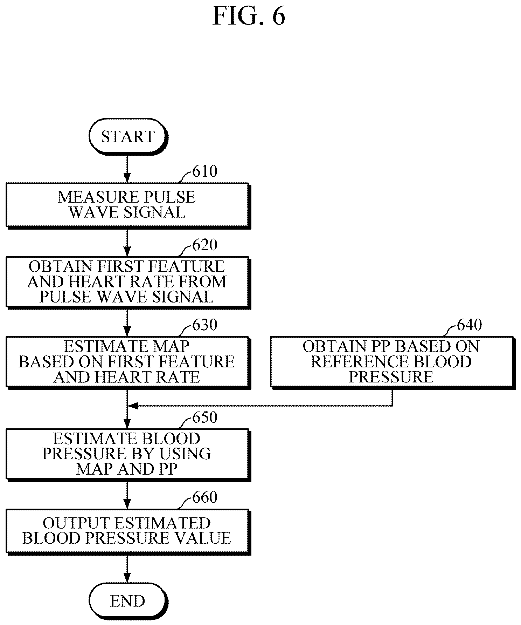

FIG. 6 is a flowchart illustrating a method of estimating blood pressure according to an example embodiment of the present disclosure. The method of FIG. 6 is an example of a method of estimating blood pressure which is performed by the apparatus for estimating blood pressure of FIGS. 1 and 5.

First, the apparatus for estimating blood pressure measures a pulse wave signal from an object in operation 610 in response to a request for estimating blood pressure.

Then, the apparatus for estimating blood pressure obtains a heart rate and a TPR feature by analyzing a waveform of the pulse wave signal in operation 620. For example, the apparatus for estimating blood pressure may obtain a plurality of unit waveforms by segmenting the waveform of the pulse wave signal, measured continuously during a predetermined period of time, into cycles and may obtain the heart rate by using the obtained plurality of unit waveforms. In addition, the apparatus for estimating blood pressure may obtain at least one representative waveform by using the plurality of unit waveforms, and may analyze the obtained representative waveform to obtain, for example, a ratio between an amplitude corresponding to a time point of the reflection wave and an amplitude corresponding to a time point of the propagation wave as the TPR feature. In particular, the time point of the reflection wave and the time point of the propagation wave may be predefined fixed time points to be commonly applied to a plurality of users, but example embodiments of the present disclosure are not limited thereto.

Subsequently, the apparatus for estimating blood pressure estimates MAP by using the obtained heart rate and the TPR feature in operation 630. For example, by normalizing the extracted heart rate and the TPR feature and by combining the normalized values, the apparatus for estimating blood pressure may obtain a variation in MAP, and may obtain the MPA based on the obtained variation in MAP and a reference MAP measured at a calibration time.

Further, the apparatus for estimating blood pressure may obtain PP based on a reference blood pressure in operation 640. For example, as described above, the apparatus for estimating blood pressure may use a reference PP at the calibration time as PP at a current time. However, the PP is not limited thereto, and the apparatus for estimating blood pressure may obtain a PP feature by using the pulse wave signal measured in operation 610, and may estimate the PP by using the obtained PP feature.

Next, the apparatus for estimating blood pressure estimates blood pressure by using the MAP and the PP in operation 650. For example, the apparatus for estimating blood pressure may independently obtain DBP and SBP based on the MAP and PP by using equations predefined for each of DBP and DBP.

Then, the apparatus for estimating blood pressure outputs the estimated blood pressure value by using various output means, such as a display, a speaker, a haptic device, etc., to provide the estimated value to a user in operation 660.

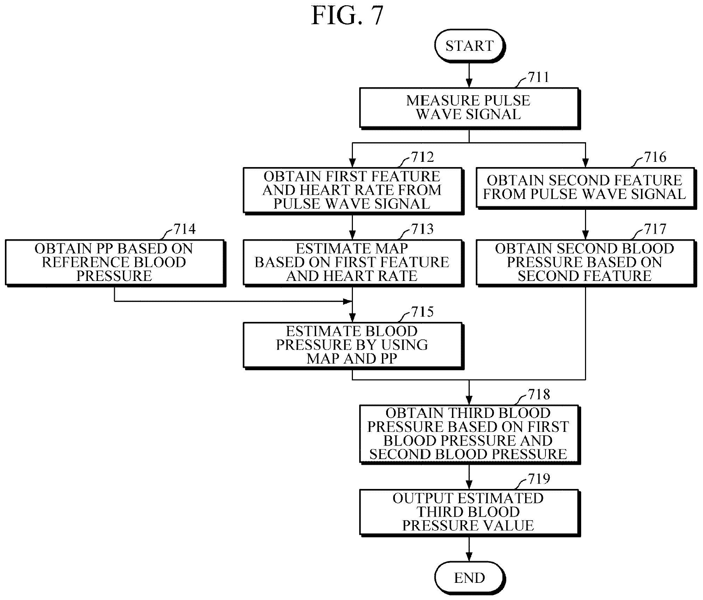

FIG. 7 is a flowchart illustrating a method of estimating blood pressure according to another example embodiment of the present disclosure. The method of FIG. 7 is an example of a method of estimating blood pressure which is performed by the apparatus for estimating blood pressure of FIG. 1 or FIG. 2, which is described in detail above, and thus will be briefly described below.

First, the apparatus for estimating blood pressure measures a pulse wave signal from an object in response to a request for estimating blood pressure in operation 711.

Then, the apparatus for estimating blood pressure obtains a first feature related to TPR and a heart rate related to CO from the measured pulse wave signal in operation 712, and estimates MAP based on the obtained first feature and heart rate in operation 713. As described above, by normalizing the extracted heart rate and the TPR feature using a heart rate and a TPR feature at the calibration time and by combining the normalized values, the apparatus for estimating blood pressure may obtain a variation in MAP, and may obtain MAP by combining the obtained variation in MAP and a reference MAP measured at the calibration time.

Further, the apparatus for estimating blood pressure obtains PP based on a reference blood pressure in operation 714, and estimates a first blood pressure in operation 715 by using the obtained PP and the MAP obtained in operation 713. As described above, the apparatus for estimating blood pressure may obtain the first blood pressure based on the MAP and PP by using a predefined first blood pressure estimation equation.

In addition, the apparatus for estimating blood pressure obtains a second feature from the obtained pulse wave signal in operation 716, and obtains a second blood pressure by using the second feature in operation 717. In particular, as described above, by analyzing a waveform of the pulse wave signal, the apparatus for estimating blood pressure obtains various characteristic point information, and obtains one or more second features, related to blood pressure, by using one or a combination of two or more of the obtained characteristic point information. Furthermore, as described above, the apparatus for estimating blood pressure may obtain a variation in the second feature by normalizing the second feature using a second feature at the calibration time, and may obtain the second blood pressure based on the variation in the second feature by using a predefined second blood pressure estimation equation.

Next, the apparatus for estimating blood pressure obtains a third blood pressure based on the first blood pressure and the second blood pressure in operation 718, and outputs the obtained third blood pressure as a final blood pressure in operation 719. According to the invention, the apparatus for estimating blood pressure evaluates reliability in estimating blood pressure based on a direction of change in the first blood pressure and a direction of change in the second blood pressure compared to the calibration time, and obtains the third blood pressure based on the first blood pressure and the second blood pressure according to a reliability evaluation result.

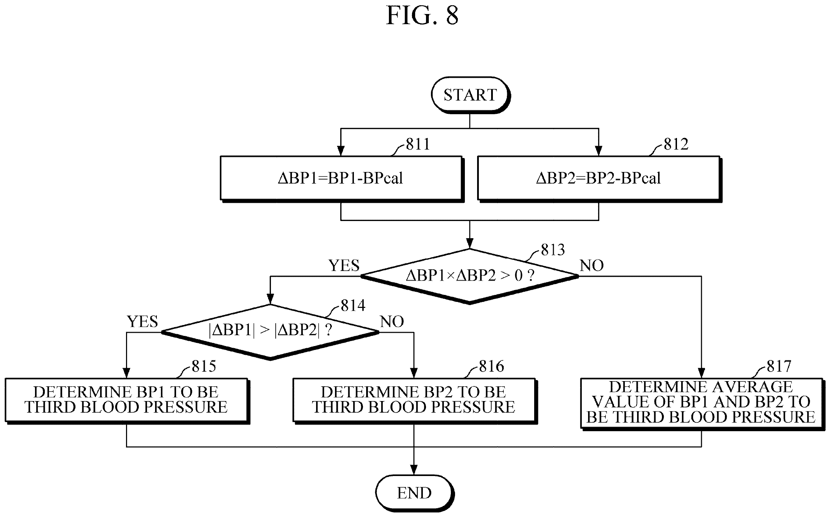

FIG. 8 is a flowchart illustrating an example of obtaining the third blood pressure in operation 718 of FIG. 7.

Referring to FIG. 8, the apparatus for estimating blood pressure may obtain a first value $\Delta BP1$, representing a change in the first blood pressure compared to the calibration time, by subtracting a reference blood pressure BPcal at the calibration time from the first blood pressure BP1 in operation 811, and may obtain a second value $\Delta BP2$, representing a change in the second blood pressure compared to the calibration time, by subtracting the reference blood pressure BPcal from the second blood pressure BP2 in operation 812.

Then, by multiplying the first value $\Delta BP1$ and the second value $\Delta BP2$, and determining whether a result value is greater than 0, the apparatus for estimating blood pressure may evaluate reliability in estimating blood pressure in operation 813.

Subsequently, upon determination, if the value obtained by multiplying the first value $\Delta BP1$ and the second value $\Delta BP2$ is greater than 0, such that the first blood pressure and the second blood pressure show the same direction of change, the apparatus for estimating blood pressure may evaluate that the reliability is high, and may compare a magnitude ($|\Delta BP1|$) of the first value and a magnitude ($|\Delta BP2|$) of the second value in operation 814 to determine one of the first value and the second value, which is greater than the other, to be the third blood pressure in operations 815 and 816. By contract, upon determination, if the value obtained by multiplying the first value $\Delta BP1$ and the second value $\Delta BP2$ in 813 is not greater than 0, such that the first blood pressure and the second blood pressure show different directions of change, the apparatus for estimating blood pressure may evaluate that the reliability is low, and may determine an average value of the first blood pressure BP1 and the second blood pressure BP2 to be the third blood pressure in operation 817. However, the example of determining the third blood pressure based on the reliability evaluation result is not limited thereto.

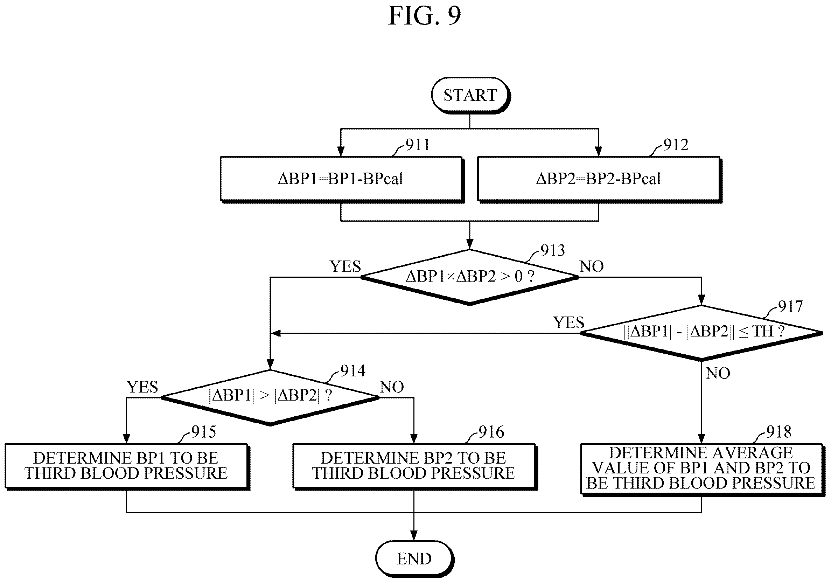

FIG. 9 is a flowchart illustrating another example of obtaining the third blood pressure in operation 718 of FIG. 7.

Referring to FIG. 9, the apparatus for estimating blood pressure may obtain a first value $\Delta BP1$ by subtracting a reference blood pressure BPcal at the calibration time from the first blood pressure BP1 in operation 911, and may obtain a second value $\Delta BP2$ by subtracting the reference blood pressure BPcal from the second blood pressure BP2 in operation 912.

Then, by multiplying the first value $\Delta BP1$ and the second value $\Delta BP2$, and determining whether a result value is greater than 0, the apparatus for estimating blood pressure may evaluate reliability in estimating blood pressure in operation 913.

Subsequently, if the value obtained by multiplying the first value $\Delta BP1$ and the second value $\Delta BP2$ is greater than 0, i.e., if the first blood pressure and the second blood pressure show the same direction of change, the apparatus for estimating blood pressure may evaluate that the reliability is high, and may compare a magnitude ($|\Delta BP1|$) of the first value and a magnitude ($|\Delta BP2|$) of the second value in operation 914 to determine one of the first value BP1 and the second value BP2, which is greater than the other, to be the third blood pressure in operations 915 and 916.

If the value obtained by multiplying the first value $\Delta BP1$ and the second value $\Delta BP2$ in 913 is not greater than 0, the apparatus for estimating blood pressure may determine whether a difference between a magnitude ($|\Delta BP1|$) of the first value and a magnitude ($|\Delta BP2|$) of the second value, i.e., an absolute value of a value obtained by subtracting the magnitude ($|\Delta BP2|$) of the second value from the magnitude ($|\Delta BP1|$) of the first value is less than or equal to a preset threshold value in 917. Upon determination in operation 917, if the difference between the magnitude ($|\Delta BP1|$) of the first value and the magnitude ($|\Delta BP2|$) of the second value is not large, the apparatus for estimating blood pressure may evaluate that the reliability is high and may perform the operations following the operation in operation 914, and if the difference between the magnitude ($|\Delta BP1|$) of the first value and the magnitude ($|\Delta BP2|$) of the second value is large, the apparatus for estimating blood pressure may evaluate that the reliability is low and may determine an average value of the first blood pressure BP1 and the second blood pressure BP2 to be the third blood pressure in operation 918.

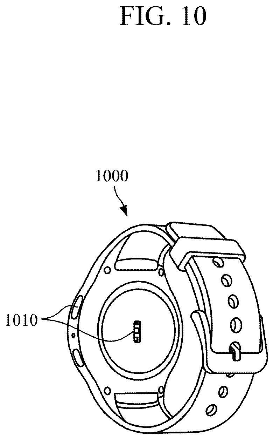

FIGS. 10 to 12 are block diagrams illustrating various structures of an electronic device including the apparatus 100 or 500 for estimating blood pressure of FIG. 1 or FIG. 5.

The electronic device includes various types of wearable devices, e.g., a smart watch, a smart band, smart glasses, smart earphones, a smart ring, a smart patch, and a smart necklace, and a mobile device such as a smartphone, a tablet PC, etc., or home appliances or various Internet of Things (IoT) devices (e.g., home IoT device, etc.) based on Internet of Things (IoT) technology.

The electronic device may include a sensor device, a processor, an input device, a communication module, a camera module, an output device, a storage device, and a power module. All the components of the electronic device may be integrally mounted in a specific device or may be distributed in two or more devices. The sensor device includes a pulse wave sensor of the apparatuses 100 and 500 for estimating blood pressure, and may further include an additional sensor, such as a gyro sensor, a Global Positioning System (GPS), and the like.

The processor may execute programs, stored in the storage device, to control components connected to the processor, and may perform various data processing or computation, including estimation of blood pressure. The processor may include a main processor, e.g., a central processing unit (CPU) or an application processor (AP), etc., and an auxiliary processor, e.g., a graphics processing unit (GPU), an image signal processor (ISP), a sensor hub processor, or a communication processor (CP), etc., which is operable independently from, or in conjunction with, the main processor.

The input device may receive a command and/or data to be used by each component of the electronic device, from a user and the like. The input device may include, for example, a microphone, a mouse, a keyboard, or a digital pen (e.g., a stylus pen, etc.).

The communication module may support establishment of a direct (e.g., wired) communication channel and/or a wireless communication channel between the electronic device and other electronic device, a server, or the sensor device within a network environment, and performing of communication via the established communication channel. The communication module may include one or more communication processors that are operable independently from the processor and supports a direct communication and/or a wireless communication. The communication module may include a wireless communication module, e.g., a cellular communication module, a short-range wireless communication module, or a global navigation satellite system (GNSS) communication module, etc., and/or a wired communication module, e.g., a local area network (LAN) communication module, a power line communication (PLC) module, and the like. These various types of communication modules may be integrated into a single chip, or may be separately implemented as multiple chips. The wireless communication module may identify and authenticate the electronic device in a communication network by using subscriber information (e.g., international mobile subscriber identity (IMSI), etc.) stored in a subscriber identification module.

The camera module may capture still images or moving images. The camera module may include a lens assembly having one mor more lenses, image sensors, image signal processors, and/or flashes. The lens assembly included in the camera module may collect light emanating from a subject to be imaged.

The output device may visually/non-visually output data generated or processed by the electronic device. The output device may include a sound output device, a display device, an audio module, and/or a haptic module.

The sound output device may output sound signals to the outside of the electronic device. The sound output device may include a speaker and/or a receiver. The speaker may be used for general purposes, such as playing multimedia or playing record, and the receiver may be used for incoming calls. The receiver may be implemented separately from, or as part of, the speaker.

The display device may visually provide information to the outside of the electronic device. The display device may include, for example, a display, a hologram device, or a projector and control circuitry to control the devices. The display device may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may obtain the sound via the input device, or may output the sound via the sound output device, and/or a speaker and/or a headphone of another electronic device directly or wirelessly connected to the electronic device.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The storage device may store driving conditions required for driving the sensor device, and various data required for other components of the electronic device. The various data may include, for example, software and input data and/or output data for a command related thereto. The storage device may include a volatile memory and/or a non-volatile memory.

The power module may manage power supplied to the electronic device. The power module may be implemented as part of, for example, a power management integrated circuit (PMIC). The power module may include a battery, which may include a primary cell which is not rechargeable, a secondary cell which is rechargeable, and/or a fuel cell.

Referring to FIG. 10, the electronic device may be implemented as a wristwatch wearable device 1000, and may include a main body and a wrist strap. A display is provided on a front surface of the main body, and may display various application screens, including time information, received message information, and the like. A sensor device 1010 may be disposed on a rear surface and/or a side surface of the main body. For example, a PPG sensor may be disposed on the rear surface, and an ECG sensor may be disposed on the side surface of the main body.

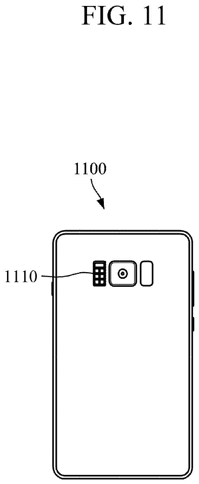

Referring to FIG. 11, the electronic device may be implemented as a mobile device 1100 such as a smartphone.

The mobile device 1100 may include a housing and a display panel. The housing may form an outer appearance of the mobile device 1100. The housing has a first surface, on which a display panel and a cover glass may be disposed sequentially, and the display panel may be exposed to the outside through the cover glass. A sensor device 1110, a camera module and/or an infrared sensor, and the like may be disposed on a second surface of the housing. The processor and various other components may be disposed in the housing.

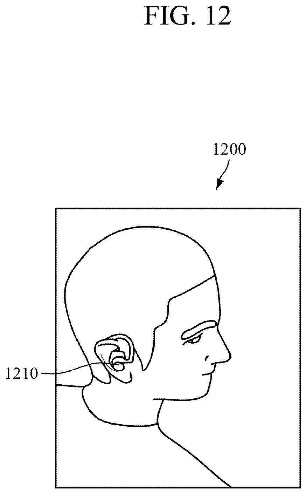

Referring to FIG. 12, the electronic device may be implemented as an ear-wearable device 1200.

The ear-wearable device 1200 may include a main body and an ear strap. A user may wear the ear-wearable device 1200 by hanging the ear strap on the auricle. The ear strap may be omitted depending on a shape of the ear-wearable device 1200. The main body may be inserted into the external auditory meatus. A sensor device 1210 may be mounted in the main body. Further, the processor may be disposed in the main body, and may estimate blood pressure by using a pulse wave signal measured by the sensor device 1210. Alternatively, the ear-wearable device 1200 may estimate blood pressure by interworking with an external device. For example, the ear-wearable device 1200 may transmit the pulse wave signal, measured by the sensor device 1210 of the ear-wearable device 1200, to an external device, e.g., a smartphone, a tablet PC, etc., through a communication module provided in the main body, so that a processor of the external device may estimate blood pressure, and may output the estimated blood pressure value through a sound output module provided in the main body of the ear-wearable device 1200.

The present disclosure can be realized as a computer-readable code written on a computer-readable recording medium or a computer-readable storage medium. The computer-readable recording medium may be any type of recording device in which data is stored in a computer-readable manner.

While not restricted thereto, an example embodiment can be embodied as computer-readable code on a computer-readable recording medium. The computer-readable recording medium is any data storage device that can store data that can be thereafter read by a computer system. Examples of the computer-readable recording medium (or the computer-readable storage medium) include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

**Claims**

1. An apparatus (100) for estimating blood pressure, comprising:

a pulse wave sensor (110) configured to measure (610, 711) a pulse wave signal from an object; and
a processor (120, 300, 400) configured to:

obtain (620, 712) a first feature and a heart rate based on the pulse wave signal, the first feature comprising a ratio between a first amplitude at a first time point and a second amplitude at a second time point in the pulse wave signal,
estimate (630, 640, 713) a mean arterial pressure, MAP, and a pulse pressure, PP, based on the first feature and the heart rate,
estimate (650, 715) a first blood pressure based on the MAP and the PP,
obtain (716) one or more second features based on the pulse wave signal, the one or more second features comprising a ratio between an amplitude at a maximum point of the pulse wave signal and an area of the pulse wave signal, and/or a ratio between an amplitude at a point of a first reflection wave and an amplitude at a point of a propagation wave among component waveforms constituting unit waveforms of the pulse wave signal, and
estimate (717) a second blood pressure by using the one or more second features,

wherein the processor (120, 300, 400) is further configured to determine reliability in estimating blood pressure based on directions of changes in the first blood pressure and the second blood pressure compared to a calibration time,
obtain (718) a third blood pressure based on the determined reliability by using at least one of the first blood pressure and the second blood pressure, and
output (719) the obtained third plod pressure as a final blood pressure.

2. The apparatus of claim 1, wherein the processor (120, 300, 400) is further configured to segment the pulse wave signal into a plurality of unit waveforms, and obtain the heart rate by using the plurality of unit waveform.

3. The apparatus of claim 1, wherein the processor (120, 300, 400) is further configured to normalize the heart rate and the first feature based on a reference heart rate and a reference first feature that are obtained at a calibration time, respectively, obtain a variation in the pulse wave signal based on the normalized heart rate and the normalized first feature, and obtain the MAP based on the variation in the pulse wave signal and a reference MAP that is measured at the calibration time.

4. The apparatus of one of claims 1 to 3, wherein the processor (120, 300, 400) is further configured to perform one or more of:

> obtaining the PP by using a reference blood pressure at a calibration time, and
> obtaining the first blood pressure by combining the MAP and the PP.

5. The apparatus of claim 4, wherein the processor (120, 300, 400) is further configured to normalize the one or more second features based on a reference second feature that is obtained at a calibration time, obtain a variation in the pulse wave signal based on the normalized one or more second features, and estimate the second blood pressure based on the variation in the pulse wave signal and a reference blood pressure at the calibration time.

6. The apparatus of claim 5, wherein upon determining that the reliability is high, the processor (120, 300, 400) is further configured to obtain one of the first blood pressure and the second blood pressure, which has a greater variation compared to a reference blood pressure that is measured at a calibration time, as the third blood pressure, and upon determining that the reliability is low, the processor (120, 300, 400) is further configured to obtain an average value of the first blood pressure and the second blood pressure as the third blood pressure.

7. The apparatus of claim 5, wherein if a first value, obtained by subtracting a reference blood pressure from the first blood pressure, has a same sign as a second value obtained by subtracting the reference blood pressure from the second blood pressure, the processor (120, 300, 400) determines that the reliability is high, and if not, the processor (120, 300, 400) determines that the reliability is low.

8. A method of estimating blood pressure, the method comprising:

> measuring (610, 711) by a pulse wave sensor (110) a pulse wave signal from an object;
> performing by a processor (120, 300, 400) the steps of:
>
> > obtaining (620, 712) a first feature and a heart rate based on the pulse wave signal, the first feature comprising a ratio between a first amplitude at a first time point and a second amplitude at a second time point in the pulse wave signal;
> > estimating (630, 640, 713) a mean arterial pressure, MAP, and a pulse pressure, PP, based on the first feature and the heart rate;

> estimating (650, 715) a first blood pressure based on the MAP and the PP;
> obtaining (716) one or more second features based on the pulse wave signal, the one or more second features comprising a ratio between an amplitude at a maximum point of the pulse wave signal and an area of the pulse wave signal, and/or a ratio between an amplitude at a point of a first reflection wave and an amplitude at a point of a propagation wave among component waveforms constituting unit waveforms of the pulse wave signal; estimating (717) a second blood pressure based on the one or more second features;
> determining reliability in estimating blood pressure based on directions of changes in the first blood pressure and the second blood pressure compared to a calibration time;
> obtaining (718) a third blood pressure based on the reliability by using at least one of the first blood pressure and the second blood pressure; and
> outputting (719) the obtained third plod pressure as a final blood pressure.

9. The method of claim 8, wherein the estimating of the MAP comprises:

> normalizing the heart rate and the first feature based on a reference heart rate and a reference first feature that are obtained at a calibration time, respectively;
> obtaining a variation in the pulse wave signal based on the normalized heart rate and the normalized first feature; and
> obtaining the MAP based on the variation in the pulse wave signal and a reference MAP that is measured at the calibration time.

10. The method of claim 8, wherein the estimating of the second blood pressure comprises:

> normalizing the obtained one or more second features based on a reference second feature that is obtained at a calibration time;
> obtaining a variation in the pulse wave signal based on the normalized one or more second features; and
> estimating the second blood pressure based on the variation in the pulse wave signal and a reference blood pressure at the calibration time.

11. The method of claim 8, wherein the obtaining of the third blood pressure comprises, in response to determining that the reliability is high, obtaining one of the first blood pressure and the second blood pressure, which has a greater variation compared to a reference blood pressure that is measured at a calibration time, as the third blood pressure, and in response to determining that the reliability is low, obtaining an average value of the first blood pressure and the second blood pressure as the third blood pressure.

12. The method of claim 8, wherein the determining of the reliability comprises, if a first value, obtained by subtracting a reference blood pressure from the first blood pressure, has a same sign as a second value obtained by subtracting the reference blood pressure from the second blood pressure, determining that the reliability is high, and if not, determining that the reliability is low.

13. A wearable device having mounted therein an apparatus for estimating blood pressure according to one of claims 1 to 7.


**Patentansprüche**

1. Vorrichtung (100) zum Schätzen von Blutdruck, umfassend:

   einen Pulswellensensor (110), konfiguriert zum Messen (610, 711) eines Pulswellensignals von einem Objekt; und
   einen Prozessor (120, 300, 400), konfiguriert zum:

   Ermitteln (620, 712) eines ersten Merkmals und einer Herzfrequenz basierend auf dem Pulswellensignal, wobei das erste Merkmal ein Verhältnis zwischen einer ersten Amplitude zu einem ersten Zeitpunkt und einer zweiten Amplitude zu einem zweiten Zeitpunkt in dem Pulswellensignal umfasst,
   Schätzen (630, 640, 713) eines mittleren arteriellen Drucks, MAP (mean arterial pressure), und eines Pulsdrucks, PP (pulse pressure), basierend auf dem ersten Merkmal und der Herzfrequenz,
   Schätzen (650, 715) eines ersten Blutdrucks basierend auf dem MAP und dem PP,
   Ermitteln (716) eines oder mehrerer zweiter Merkmale basierend auf dem Pulswellensignal, wobei das eine oder die mehreren zweiten Merkmale ein Verhältnis zwischen einer Amplitude an einem Maximalpunkt des Pulswellensignals und einer Fläche des Pulswellensignals und/oder ein Verhältnis zwischen einer Amplitude an einem Punkt einer ersten Reflexionswelle und einer Amplitude an einem Punkt einer Ausbreitungswelle unter Komponentenwellenformen, die Einheitswellenformen des Pulswellensignals bilden, umfassen, und
Schätzen (717) eines zweiten Blutdrucks unter Verwendung des einen oder der mehreren zweiten Merkmale,

wobei der Prozessor (120, 300, 400) ferner konfiguriert ist, zum Bestimmen einer Zuverlässigkeit beim Schätzen des Blutdrucks basierend auf Richtungen von Änderungen des ersten Blutdrucks und des zweiten Blutdrucks im Vergleich zu einer Kalibrierungszeit,
Ermitteln (718) eines dritten Blutdrucks basierend auf der bestimmten Zuverlässigkeit unter Verwendung von mindestens einem von dem ersten Blutdruck und dem zweiten Blutdruck, und
Ausgeben (719) des ermittelten dritten Blutdrucks als endgültigen Blutdruck.

2. Vorrichtung nach Anspruch 1, wobei der Prozessor (120, 300, 400) ferner konfiguriert ist, zum Segmentieren des Pulswellensignals in eine Vielzahl von Einheitswellenformen und zum Ermitteln der Herzfrequenz unter Verwendung der Vielzahl von Einheitswellenformen.

3. Vorrichtung nach Anspruch 1, wobei der Prozessor (120, 300, 400) ferner konfiguriert ist, zum Normieren der Herzfrequenz und des ersten Merkmals basierend auf einer ReferenzHerzfrequenz und einem ersten Referenzmerkmal, die jeweils zu einem Kalibrierungszeitpunkt ermittelt werden, zum Ermitteln einer Variation in dem Pulswellensignal basierend auf der normierten Herzfrequenz und dem normierten ersten Merkmal, und zum Ermitteln des MAP basierend auf der Variation in dem Pulswellensignal und einem Referenz-MAP, der zu dem Kalibrierungszeitpunkt gemessen wird.

4. Vorrichtung nach einem der Ansprüche 1 bis 3, wobei der Prozessor (120, 300, 400) ferner konfiguriert ist, zum Durchführen eines oder mehrerer der folgenden Schritte:

   Ermitteln des PP unter Verwendung eines Referenzblutdrucks zu einem Kalibrierungszeitpunkt, und
   Ermitteln des ersten Blutdrucks durch Kombinieren des MAP und des PP.

5. Vorrichtung nach Anspruch 4, wobei der Prozessor (120, 300, 400) ferner konfiguriert ist, zum Normieren des einen oder der mehreren zweiten Merkmale basierend auf einem zweiten Referenzmerkmal, das zu einem Kalibrierungszeitpunkt ermittelt wird, zum Ermitteln einer Variation des Pulswellensignals basierend auf dem normierten einen oder den mehreren zweiten Merkmalen, und zum Schätzen des zweiten Blutdrucks basierend auf der Variation des Pulswellensignals und einem Referenzblutdruck zu dem Kalibrierungszeitpunkt.

**6.** Vorrichtung nach Anspruch 5, wobei bei der Bestimmung, dass die Zuverlässigkeit hoch ist, der Prozessor (120, 300, 400) ferner konfiguriert ist, zum Ermitteln eines von dem ersten Blutdruck und dem zweiten Blutdruck, welcher eine größere Variation im Vergleich zu einem Referenzblutdruck hat, der zu einem Kalibrierungszeitpunkt gemessen wird, als den dritten Blutdruck, und bei der Bestimmung, dass die Zuverlässigkeit niedrig ist, der Prozessor (120, 300, 400) ferner konfiguriert ist, zum Ermitteln eines Durchschnittswertes des ersten Blutdrucks und des zweiten Blutdrucks als den dritten Blutdruck.

**7.** Vorrichtung nach Anspruch 5, wobei, wenn ein erster Wert, der durch Subtraktion eines Referenzblutdrucks von dem ersten Blutdruck ermittelt wird, dasselbe Vorzeichen hat wie ein zweiter Wert, der durch Subtraktion des Referenzblutdrucks von dem zweiten Blutdruck ermittelt wird, der Prozessor (120, 300, 400) bestimmt, dass die Zuverlässigkeit hoch ist, und wenn nicht, der Prozessor (120, 300, 400) bestimmt, dass die Zuverlässigkeit niedrig ist.

**8.** Verfahren zum Schätzen von Blutdruck, wobei das Verfahren umfasst:

Messen (610, 711) eines Pulswellensignals von einem Objekt durch einen Pulswellensensor (110);
Durchführen der folgenden Schritte durch einen Prozessor (120, 300, 400):

Ermitteln (620, 712) eines ersten Merkmals und einer Herzfrequenz basierend auf dem Pulswellensignal, wobei das erste Merkmal ein Verhältnis zwischen einer ersten Amplitude zu einem ersten Zeitpunkt und einer zweiten Amplitude zu einem zweiten Zeitpunkt in dem Pulswellensignal umfasst;
Schätzen (630, 640, 713) eines mittleren arteriellen Drucks, MAP (mean arterial pressure), und eines Pulsdrucks, PP (pulse pressure), basierend auf dem ersten Merkmal und der Herzfrequenz;
Schätzen (650, 715) eines ersten Blutdrucks basierend auf dem MAP und dem PP;
Ermitteln (716) eines oder mehrerer zweiter Merkmale basierend auf dem Pulswellensignal, wobei das eine oder die mehreren zweiten Merkmale ein Verhältnis zwischen einer Amplitude an einem Maximalpunkt des Pulswellensignals und einer Fläche des Pulswellensignals und/oder ein Verhältnis zwischen einer Amplitude an einem Punkt einer ersten Reflexionswelle und einer Amplitude an einem Punkt einer Ausbreitungswelle unter Komponentenwellenformen, die Einheitswellenformen des Pulswellensignals bilden, umfassen;
Schätzen (717) eines zweiten Blutdrucks basierend auf dem einen oder mehreren zweiten Merkmalen;
Bestimmen einer Zuverlässigkeit beim Schätzen des Blutdrucks basierend auf Richtungen von Änderungen des ersten Blutdrucks und des zweiten Blutdrucks im Vergleich zu einer Kalibrierungszeit;
Ermitteln (718) eines dritten Blutdrucks basierend auf der Zuverlässigkeit unter Verwendung von mindestens einem von dem ersten Blutdruck und dem zweiten Blutdruck; und
Ausgeben (719) des ermittelten dritten Blutdrucks als einen endgültigen Blutdruck.

**9.** Verfahren nach Anspruch 8, wobei das Schätzen des MAP umfasst:

Normieren der Herzfrequenz und des ersten Merkmals basierend auf einer Referenzherzfrequenz und einem ersten Referenzmerkmal, die jeweils zu einem Kalibrierungszeitpunkt ermittelt werden;
Ermitteln einer Variation in dem Pulswellensignal basierend auf der normierten Herzfrequenz und dem normierten ersten Merkmal; und
Ermitteln des MAP basierend auf der Variation in dem Pulswellensignal und einem Referenz-MAP, der zum Kalibrierungszeitpunkt gemessen wird.

**10.** Verfahren nach Anspruch 8, wobei das Schätzen des zweiten Blutdrucks umfasst:

Normieren des ermittelten einen oder der mehreren zweiten Merkmale basierend auf einem zweiten Referenzmerkmal, das zu einem Kalibrierungszeitpunkt ermittelt wird;
Ermitteln einer Variation in dem Pulswellensignal basierend auf dem normierten einen oder den mehreren zweiten Merkmalen; und
Schätzen des zweiten Blutdrucks basierend auf der Variation in dem Pulswellensignal und einem Referenzblutdruck zum Kalibrierungszeitpunkt.

**11.** Verfahren nach Anspruch 8, wobei das Ermitteln des dritten Blutdrucks umfasst, in Reaktion auf das Bestimmen, dass die Zuverlässigkeit hoch ist, Ermitteln eines von dem ersten Blutdruck und dem zweiten Blutdruck, welcher eine größere Variation im Vergleich zu einem Referenzblutdruck aufweist, der zu einem Kalibrierungszeitpunkt gemessen wird, als den dritten Blutdruck, und in Reaktion auf das Bestimmen, dass die Zuverlässigkeit niedrig ist, Ermitteln eines Durchschnittswertes des ersten Blutdrucks und des zweiten Blutdrucks als den dritten Blutdruck.

**12.** Verfahren nach Anspruch 8, wobei das Bestimmen der Zuverlässigkeit umfasst, wenn ein erster Wert, der durch Subtraktion eines Referenzblutdrucks von dem ersten Blutdruck ermittelt wird, dasselbe Vorzeichen wie ein zweiter Wert hat, der durch Subtraktion des Referenzblutdrucks von dem zweiten Blutdruck ermittelt wird, das Bestimmen, dass die Zuverlässigkeit hoch ist, und wenn nicht, das Bestimmen, dass die Zuverlässigkeit niedrig ist.

**13.** Tragbares Gerät mit einer darin angebrachten Vorrichtung zum Schätzen von Blutdruck nach einem der Ansprüche 1 bis 7.


**Revendications**

**1.** Appareil (100) pour estimer une pression sanguine, comprenant :

un capteur d'onde d'impulsion (110) configuré pour mesurer (610, 711) un signal d'onde d'impulsion issu d'un objet ; et
un processeur (120, 300, 400) configuré pour :

obtenir (620, 712) une première caractéristique et une fréquence cardiaque reposant sur le signal d'onde d'impulsion, la première caractéristique comprenant un rapport entre une première amplitude à un premier point temporel et une deuxième amplitude à un deuxième point temporel dans le signal d'onde d'impulsion,
estimer (630, 640, 713) une pression artérielle moyenne, MAP (Mean Arterial Pressure), et une pression de pouls (PP, Pulse Pressure), selon la première caractéristique et la fréquence cardiaque,
estimer (650, 715) une première pression sanguine reposant sur la MAP et la PP,
obtenir (716) une ou plusieurs deuxièmes caractéristiques reposant sur le signal d'onde d'impulsion, l'une ou plusieurs deuxièmes caractéristiques comprenant un rapport entre une amplitude à un point maximal du signal d'onde d'impulsion et une zone du signal d'onde d'impulsion, et/ou un rapport entre une amplitude à un point d'une première onde de réflexion et une amplitude à un point d'une onde de propagation parmi des formes d'onde composantes constituant des formes d'onde d'unité du signal d'onde d'impulsion, et
estimer (717) une deuxième pression sanguine en utilisant les une ou plusieurs deuxièmes caractéristiques,
dans lequel le processeur (120, 300, 400) est configuré en outre pour déterminer une fiabilité dans une estimation de pression sanguine selon des directions de changements dans la première pression sanguine et la deuxième pression sanguine comparées à un moment d'étalonnage,
obtenir (718) une troisième pression sanguine selon la fiabilité déterminée en utilisant au moins une de la première pression sanguine et de la deuxième pression sanguine, et
produire en sortie (719) la troisième pression sanguine obtenue en tant que pression sanguine finale.

**2.** L'appareil de la revendication 1, dans lequel le processeur (120, 300, 400) est configuré en outre pour segmenter le signal d'onde d'impulsion en une pluralité de formes d'onde d'unité, et obtenir la fréquence cardiaque en utilisant la pluralité de formes d'onde d'unité.

**3.** L'appareil de la revendication 1, dans lequel le processeur (120, 300, 400) est configuré en outre pour normaliser la fréquence cardiaque et la première caractéristique selon une fréquence cardiaque de référence et une première caractéristique de référence qui sont obtenues à un moment d'étalonnage, respectivement, obtenir une variation dans le signal d'onde d'impulsion selon la fréquence cardiaque normalisée et la première caractéristique normalisée, et obtenir la MAP selon la variation dans le signal d'onde d'impulsion et une MAP de référence qui est mesurée au moment d'étalonnage.

**4.** L'appareil de l'une des revendications 1 à 3, dans lequel le processeur (120, 300, 400) est configuré en outre pour exécuter une ou plusieurs des opérations suivantes :

une obtention de la PP en utilisant une pression sanguine de référence à un moment d'étalonnage, et
une obtention de la première pression sanguine en combinant la MAP et la PP.

**5.** L'appareil de la revendication 4, dans lequel le processeur (120, 300, 400) est configuré en outre pour normaliser l'une ou plusieurs deuxièmes caractéristiques selon une deuxième caractéristique de référence qui est obtenue à un moment d'étalonnage, obtenir une variation dans le signal d'onde d'impulsion selon l'une ou plusieurs deuxièmes caractéristiques normalisées, et estimer la deuxième pression sanguine selon la variation dans le signal d'onde d'impulsion et une pression sanguine de référence au moment d'étalonnage.

6. L'appareil de la revendication 5, dans lequel sur détermination que la fiabilité est élevée, le processeur (120, 300, 400) est configuré en outre pour obtenir une de la première pression sanguine et de la deuxième pression sanguine, qui présente une variation plus élevée comparée à une pression sanguine de référence qui est mesurée à un moment d'étalonnage, comme la troisième pression sanguine, et sur détermination que la fiabilité est faible, le processeur (120, 300, 400) est configuré en outre pour obtenir une valeur moyenne de la première pression sanguine et de la deuxième pression sanguine comme la troisième pression sanguine.

7. L'appareil de la revendication 5, dans lequel si une première valeur, obtenue en soustrayant une pression sanguine de référence de la première pression sanguine, présente un même signe qu'une deuxième valeur obtenue en soustrayant la pression sanguine de référence de la deuxième pression sanguine, le processeur (120, 300, 400) détermine que la fiabilité est élevée, et dans la négative, le processeur (120, 300, 400) détermine que la fiabilité est faible.

8. Procédé d'estimation de pression sanguine, le procédé comprenant :

   une mesure (610, 711) par un capteur d'onde d'impulsion (110) d'un signal d'onde d'impulsion issu d'un objet ;
   une exécution par un processeur (120, 300, 400) des étapes suivantes :

   une obtention (620, 712) d'une première caractéristique et d'une fréquence cardiaque reposant sur le signal d'onde d'impulsion, la première caractéristique comprenant un rapport entre une première amplitude à un premier point temporel et une deuxième amplitude à un deuxième point temporel dans le signal d'onde d'impulsion ;
   une estimation (630, 640, 713) d'une pression artérielle moyenne, MAP (Mean Arterial Pressure), et d'une pression de pouls (PP, Pulse Pressure), selon la première caractéristique et la fréquence cardiaque ;
   une estimation (650, 715) d'une première pression sanguine reposant sur la MAP et la PP ;
une obtention (716) d'une ou plusieurs deuxièmes caractéristiques reposant sur le signal d'onde d'impulsion, l'une ou plusieurs deuxièmes caractéristiques comprenant un rapport entre une amplitude à un point maximal du signal d'onde d'impulsion et une zone du signal d'onde d'impulsion, et/ou d'un rapport entre une amplitude à un point d'une première onde de réflexion et une amplitude à un point d'une onde de propagation parmi des formes d'onde composantes constituant des formes d'onde d'unité du signal d'onde d'impulsion ;
une estimation (717) d'une deuxième pression sanguine selon les une ou plusieurs deuxièmes caractéristiques ;
une détermination d'une fiabilité dans une estimation de pression sanguine selon des directions de changements dans la première pression sanguine et la deuxième pression sanguine comparées à un moment d'étalonnage ;
une obtention (718) d'une troisième pression sanguine selon la fiabilité déterminée en utilisant au moins une de la première pression sanguine et de la deuxième pression sanguine ; et
une production en sortie (719) de la troisième pression sanguine obtenue en tant que pression sanguine finale.

9. Le procédé de la revendication 8, dans lequel l'estimation de la MAP comprend :

   une normalisation de la fréquence cardiaque et de la première caractéristique selon une fréquence cardiaque de référence et une première caractéristique de référence qui sont obtenues à un moment d'étalonnage, respectivement ;
   une obtention d'une variation dans le signal d'onde d'impulsion selon la fréquence cardiaque normalisée et la première caractéristique normalisée ; et
   une obtention de la MAP selon la variation dans le signal d'onde d'impulsion et une MAP de référence qui est mesurée au moment d'étalonnage.

10. Le procédé de la revendication 8, dans lequel l'estimation de la deuxième pression sanguine comprend :

    une normalisation de l'une ou plusieurs deuxièmes caractéristiques obtenues selon une deuxième caractéristique de référence qui est obtenue à un moment d'étalonnage ;

une obtention d'une variation dans le signal d'onde d'impulsion selon les une ou plusieurs deuxièmes caractéristiques normalisées ; et

une estimation de la deuxième pression sanguine selon la variation dans le signal d'onde d'impulsion et une pression sanguine de référence au moment d'étalonnage.

11. Le procédé de la revendication 8, dans lequel l'obtention de la troisième pression sanguine comprend, en réaction à une détermination que la fiabilité est élevée, une obtention d'une de la première pression sanguine et de la deuxième pression sanguine, qui présente une variation plus élevée comparée à une pression sanguine de référence qui est mesurée à un moment d'étalonnage, comme la troisième pression sanguine, et en réaction à une détermination que la fiabilité est faible, une obtention d'une valeur moyenne de la première pression sanguine et de la deuxième pression sanguine comme la troisième pression sanguine.

12. Le procédé de la revendication 8, dans lequel la détermination de la fiabilité comprend, si une première valeur, obtenue en soustrayant une pression sanguine de référence de la première pression sanguine, présente un même signe qu'une deuxième valeur obtenue en soustrayant la pression sanguine de référence de la deuxième pression sanguine, une détermination que la fiabilité est élevée, et dans la négative, une détermination que la fiabilité est faible.

13. Dispositif vestimentaire présentant, monté sur celui-ci, un appareil pour estimer une pression sanguine selon l'une des revendications 1 à 7.

FIG. 1

FIG. 2

EP 4 137 043 B1

# FIG. 3

EP 4 137 043 B1

## FIG. 4

# FIG. 5

# FIG. 6

```
              ┌─────────┐
              │  START  │
              └─────────┘
                   │
                   ▼            ╱610
        ┌──────────────────────┐
        │   MEASURE PULSE       │
        │    WAVE SIGNAL        │
        └──────────────────────┘
                   │
                   ▼            ╱620
        ┌──────────────────────┐
        │ OBTAIN FIRST FEATURE  │
        │ AND HEART RATE FROM   │
        │  PULSE WAVE SIGNAL    │
        └──────────────────────┘
                   │
                   ▼            ╱630                           ╱640
        ┌──────────────────────┐          ┌──────────────────────┐
        │    ESTIMATE MAP       │          │  OBTAIN PP BASED ON   │
        │ BASED ON FIRST FEATURE│          │  REFERENCE BLOOD      │
        │   AND HEART RATE      │          │     PRESSURE          │
        └──────────────────────┘          └──────────────────────┘
                   │                                  │
                   ▼◄─────────────────────────────────┘
                                ╱650
        ┌──────────────────────┐
        │   ESTIMATE BLOOD      │
        │ PRESSURE BY USING     │
        │    MAP AND PP         │
        └──────────────────────┘
                   │
                   ▼            ╱660
        ┌──────────────────────┐
        │  OUTPUT ESTIMATED     │
        │ BLOOD PRESSURE VALUE  │
        └──────────────────────┘
                   │
                   ▼
              ┌─────────┐
              │   END   │
              └─────────┘
```

## FIG. 7

```
                        ( START )
                            │
                            │  ┌711
                            ▼
              ┌──────────────────────────┐
              │      MEASURE PULSE        │
              │       WAVE SIGNAL         │
              └──────────────────────────┘
                     │              │
          ┌712       ▼              ▼      ┌716
  ┌────────────────────────┐  ┌──────────────────────────┐
  │   OBTAIN FIRST FEATURE  │  │   OBTAIN SECOND FEATURE   │
  │   AND HEART RATE FROM    │  │  FROM PULSE WAVE SIGNAL    │
  │    PULSE WAVE SIGNAL     │  └──────────────────────────┘
  └────────────────────────┘              │
                  │                         │  ┌717
        ┌713      ▼                         ▼
┌──714─────────┐ ┌──────────────┐  ┌──────────────────────┐
│OBTAIN PP BASED│ │ ESTIMATE MAP │  │ OBTAIN SECOND BLOOD  │
│ON REFERENCE   │ │BASED ON FIRST│  │  PRESSURE BASED ON   │
│BLOOD PRESSURE │ │FEATURE AND   │  │    SECOND FEATURE    │
└──────────────┘ │HEART RATE    │  └──────────────────────┘
         │        └──────────────┘
         │              │
         └──────────────┤  ┌715
                        ▼
              ┌──────────────────┐
              │  ESTIMATE BLOOD   │
              │ PRESSURE BY USING │
              │    MAP AND PP     │
              └──────────────────┘
                        │
                        │              ┌718
                        ▼
              ┌──────────────────────────┐
              │    OBTAIN THIRD BLOOD     │
              │  PRESSURE BASED ON FIRST  │
              │    BLOOD PRESSURE AND     │
              │  SECOND BLOOD PRESSURE    │
              └──────────────────────────┘
                        │
                        │  ┌719
                        ▼
              ┌──────────────────┐
              │  OUTPUT ESTIMATED │
              │    THIRD BLOOD    │
              │  PRESSURE VALUE   │
              └──────────────────┘
                        │
                        ▼
                    ( END )
```

EP 4 137 043 B1

# FIG. 8

START

ΔBP1=BP1-BPcal  (811)

ΔBP2=BP2-BPcal  (812)

ΔBP1×ΔBP2 > 0 ?  (813)
— YES / NO

|ΔBP1| > |ΔBP2| ?  (814)
— YES / NO

DETERMINE BP1 TO BE THIRD BLOOD PRESSURE  (815)

DETERMINE BP2 TO BE THIRD BLOOD PRESSURE  (816)

DETERMINE AVERAGE VALUE OF BP1 AND BP2 TO BE THIRD BLOOD PRESSURE  (817)

END

## FIG. 9

START

911 — $\Delta BP1 = BP1 - BPcal$

912 — $\Delta BP2 = BP2 - BPcal$

913 — $\Delta BP1 \times \Delta BP2 > 0$ ?

YES / NO

917 — $\|\Delta BP1\| - |\Delta BP2\| \leq TH$ ?

YES / NO

914 — $|\Delta BP1| > |\Delta BP2|$ ?

YES / NO

915 — DETERMINE BP1 TO BE THIRD BLOOD PRESSURE

916 — DETERMINE BP2 TO BE THIRD BLOOD PRESSURE

918 — DETERMINE AVERAGE VALUE OF BP1 AND BP2 TO BE THIRD BLOOD PRESSURE

END

# FIG. 10

# FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3613340 A1
- EP 3632302 A1